# EUROPEAN PATENT APPLICATION

(11) **EP 1 520 559 A1**
(43) Date of publication of application: **06.04.2005**
(21) Application number: 03022172.5
(22) Date of filing: 30.09.2003
(51) Int. Cl.: A61F 2/34

(54) **Acetubular liner**

(71) Applicant: Technische Universität München, 80333 München (DE)
(72) Inventor: Bader, Rainer, Dr. med., Dipl.-Ing., 73102 Birenbach (DE); Bernhart, Michael, 80335 München (DE); Mittelmeier, Wolfram, PD Dr. med., 81735 München (DE); Steinhauser, Erwin, Dipl.-Ing., 85560 Ebersberg (DE)
(74) Representative: VOSSIUS & PARTNER

(57) **Abstract**

The present invention is directed to an implant for total hip joint replacement and a method for manufacturing such implant. In particular, the present invention relates to improved acetabular liners, total hip prosthetic assemblies comprising such liners and methods for manufacturing such liners.

## Description

The present invention is directed to an implant for total hip joint replacement and a method for manufacturing such implant. In particular, the present invention relates to improved acetabular liners, total hip prosthetic assemblies comprising such liners and methods for manufacturing such liners.

Prostheses or artificial implants are widely accepted and commonly used for replacement of all or a portion of a diseased or injured human hip joint. The human hip joint is a multi-axial joint of ball-and-socket type, wherein the generally ball-shaped head of the femur is positioned within the socket-shaped acetabulum of the pelvis. In a total hip joint replacement, both the femoral head and the surface of the acetabulum are replaced with prosthetic devices or implants. Hence, an assembly for total hip replacement essentially comprises a femoral component and acetabular components. The acetabular components generally comprise an acetabular cup component (or shell) and an acetabular liner to be fitted within the cup component, but in some cases may comprise the liner alone.

Although femoral and acetabular components are commonly used to restore function of the hip joint, there are several problems associated with their use. Apart from improper surgical procedures and inadequate positioning of these implant components, the design of these components is one of the main factors responsible for damage of the liner or, even more important, for a dislocation, i.e. a dislocation of the head of the femoral component from the acetabular liner. For example, subluxation and dislocation may be caused by impingement, i.e. contact between the neck of the femoral component and the rim surface of the acetabular liner. Due to an insufficient range of motion of the total hip replacement dislocation can occur when the neck of the femoral component is pressed against the edge or rim surface of the liner such that, the edge or rim surface of the liner acts as a fulcrum about which the femoral component pivots, thereby causing the head fixed on the femoral component to dislocate from its position within the liner. For at least this reason, stable containment of the femoral head within the cavity of the acetabular liner has been a problem frequently associated with several standard acetabular liners. Since such dislocation generally causes significant pain to the patient and leads to further costs associated with readjusting or replacing the hip joint components, there is a need to address this problem.

In order to address this problem, a wide variety of acetabular liners have been designed to provide a more secure containment of the femoral head. For example, acetabular liners have been designed having partially or fully elevated rims in order to encompass a greater surface of the femoral head. Such acetabular liners are disclosed in EP-A-0 556 926 and WO 02/09615. Although these type of liners have proven to provide greater stability, the range of motion of the femoral component relative to the liner is significantly reduced compared to standard non-elevated liners. A reduced range of motion can be a significant disadvantage to fairly active patients as far as physical activities are concerned.

Therefore, there is a need for an acetabular liner which provides secure containment of the femoral head while also allowing a range of motion similar to that achieved using standard non-elevated liners.

To this end, a variety of approaches have been used to provide secure containment of the femoral head within the acetabular liner while allowing the relatively broad range of motion provided with the non-elevated liners. WO 01/67999 discloses a method of producing an acetabular liner in which the rim surface geometry varies, rather than being set, in order to optimise the range of motion and minimize interference with the neck of the femoral component. The variable rim surface is employed around the edge of the internal concave surface of the liner, i.e. around the circumference of the inside diameter of a generally hemispheric acetabular liner. The variable rim surface is achieved by having a chamfer with a variety of specific angles. Another approach is disclosed in WO 02/058597, which describes a containment system suitable for constraining a femoral head within an acetabular liner. The acetabular liner has a cavity and an opening having a rim surface, which is provided with a web along a portion of it's surface. Also, the head of a femoral component is designed to have a cooperating surface that corresponds with the web of the acetabular liner such that when the cooperating surface of the femoral head is aligned with the web of the liner, the femoral head may be inserted into the liner. Once the femoral head is rotated and the femoral stem component attached, the femoral head is constrained within the cavity of the liner thereby preventing dislocation of the head. Hence, using the approach of WO 02/058597, both the acetabular liner and the femoral component have to be provided with the web portion and corresponding cooperating surface, respectively.

An object of the present invention is to provide improved acetabular liners which are simple in construction and/or overcome the aforementioned disadvantages of the prior art as well as to provide methods for manufacturing improved acetabular liners and assemblies for total hip joint replacement comprising improved liners.

This object is achieved with the features of the claims. The present invention is directed to acetabular liners as recited in claims 1 to 12, methods of manufacturing acetabular liners or implants used in assemblies for total hip joint replacement as recited in claims 13 and 14, and an endoprosthetic assembly for total hip joint replacement as recited in claim 15.

Preferred embodiments of the acetabular liner of the present invention will be further described by the following description and drawings:
- **Figure 1**: is a perspective view of an embodiment of the acetabular liner of the present invention.
- **Figure 2**: is a cross-sectional view of the embodiment shown in Figure 1 taken at the cross-section A-A indicated in Figure 3.
- **Figure 3**: is a top view of the embodiment of the present invention shown in Figure 1.
- **Figure 4**: is a cross-sectional longitudinal side view of a first embodiment of the acetabular liner of the present invention.
- **Figure 5**: is a cross-sectional longitudinal side view of a second embodiment of the acetabular liner of the present invention.
- **Figure 6**: is a cross-sectional longitudinal side view of a third embodiment of the acetabular liner of the present invention.

As can be seen by reference to the drawings, the acetabular liner of the present invention is designated generally by the reference number 10. The acetabular liner 10 comprises essentially an outer surface, an inner surface being concave and forming a cavity 20, and a rim surface. The cavity 20 is adapted to receive a femoral head fixed on a stem component. The rim surface is located circumferentially along the top edge of the liner and joins upper edges of the inner and outer surfaces. The rim surface also has at least one elevated portion 30 and at least one non-elevated portion 40.

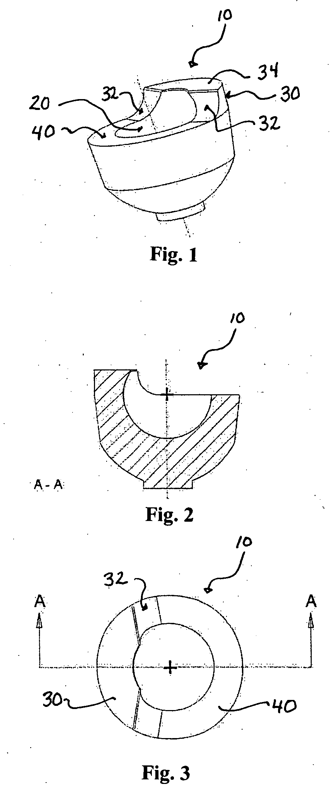

In an aspect of the acetabular liner of the present invention, the at least one elevated portion 30 has a transitional area 32 at each end, as shown in Figures 1 to 6. The transitional area 32 connects the top surface 34 of the elevated portion 30 and the non-elevation portion 40 of the rim surface. In this aspect of the present invention, at least a portion of the transitional area 32 is concave for allowing an increased range of motion of the stem component relative to the acetabular liner.

It was found that, although the acetabular liner of the present invention has at least one elevated portion designed to provide improved containment of the femoral head, the concave structure of the transitional area enabled the acetabular liner of the present invention to allow the femoral stem component approximately the same range of motion for physiological hip movements of the patients as that allowed with standard non-elevated liners. Hence, greater stability is attained without a significant decrease in the range of motion compared to standard non-elevated liners.

The shape of the transitional area 32 can also be made to correspond to the cross-sectional shape of the neck attached to the femoral head of the femoral stem component.

The transitional area 32 can also be curved with a radius of curvature. Preferably, the radius of curvature is greater than the radius of the neck attached to the femoral head of the femoral stem component.

In another aspect of the present invention, the elevated portion 30 of the acetabular liner 10 forms approximately one third or less of the circumferential rim surface.

In yet another aspect of the present invention, the elevated portion 30 of the acetabular liner 10 has a concave inner surface that is a continuation of the inner surface of the cavity 20 of the liner thereby serving to,securely contain the femoral head of a stem component in a snap fit.

The aforementioned aspects of the present invention may be also combined to form different embodiments, and each of the embodiments may comprise the following specific embodiments including further features of the acetabular liner of the present invention.

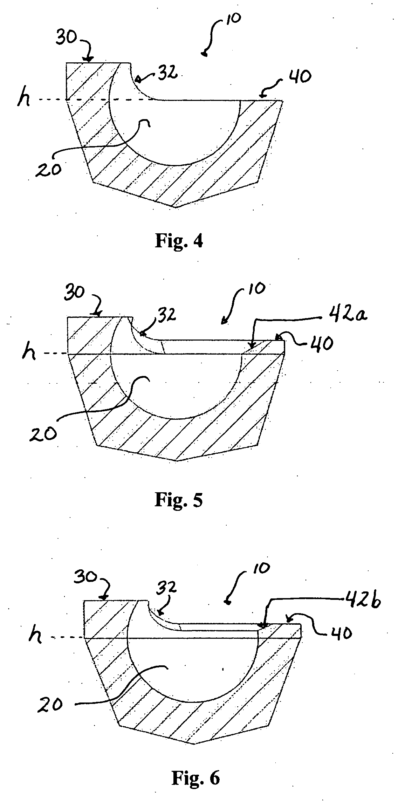

In a first embodiment of the acetabular liner of the present invention, the non-elevated portion 40 of the rim surface is approximately at the same level of horizontal axis *h* located at or near the hemisphere of the cavity 20 (hereafter referred to as hemisphere axis *h*), as shown in Figure 4. Further the fulcrum of the femoral head is located on the axis *h*.

The non-elevated portion 40 of the rim surface may also extend at least in part above the hemisphere axis *h* of the cavity 20. For example, in a second embodiment of the acetabular liner 10 of the present invention as shown in Figure 5, the non-elevated portion 40 of the rim surface extends above the hemisphere axis *h* and has a bevelled surface 42a. In this embodiment, the bevelled surface 42a is so bevelled to extend approximately to the hemisphere axis *h* of the cavity 20 such that, when a femoral component is moved to an extreme position, the neck fixed to the femoral head of the femoral component first contacts an inner edge of the bevelled surface 42a and then contacts the entire bevelled surface 42a. This provides an initial impingement at the inner edge and then full contact on the entire surface thereby preventing material damage to or material flow within the liner. The initial arrest at the inner edge of the liner also provides a dampening effect which serves to diminish the amount of contact forces against the rim surface thereby minimizing damage to the liner. The bevelled surface 42a can also be made to conform with the shape of the neck attached to the femoral head of the femoral component in order to enhance full contact between the neck of the femoral component and the entire bevelled surface 42a when the femoral component is moved to an extreme position located approximately at the limit of the range of motion, of the femoral component within the liner 10.

In a third embodiment of the acetabular liner 10 of the present invention as shown in Figure 6, the non-elevated portion 40 of the rim surface also extends above the hemisphere axis *h* of the cavity 20 and has a bevelled surface 42b. In this embodiment, the bevelled surface 42b of the non-elevated portion 40 is so formed that an improved snap fit between the femoral head and the liner 10 is provided over the entire circumference of the cavity 20. This snap fit also enables a more secure containment of the femoral head within the liner thereby providing greater stability against dislocation.

The various aspects and embodiments of the acetabular liner of the present invention may also further comprise means for attaching the liner securely to an acetabular cup component. For example, the outer surface of the acetabular liner 10 may have a means for securely fixing the acetabular liner 10 within an acetabular cup component.

The acetabular liner of the present invention may be manufactured from a variety of materials including, but not limited to, ceramic and polyethylene materials. Preferably, the acetabular liner of the present invention is manufactured from an ultra high molecular weight polyethylene (UHMW-PE) or cross-linked polyethylene.

The present invention is also directed to an assembly for total hip joint replacement comprising an acetabular liner 10, an acetabular cup component adapted to receive and be attached to the acetabular liner 10, and a femoral component comprising a head, neck and stem, wherein the head is adapted to articulate within the cavity 20 of the acetabular liner 10.

In another aspect, the present invention is directed to a method for manufacturing an implant, in particular an acetabular liner 10, used in an assembly for total hip joint replacement. The method of the present invention comprises: providing an outer surface; providing an inner surface being concave; forming a cavity using the inner surface, the cavity being adapted to receive a femoral head fixed on a femoral stem component; providing a rim surface circumferentially along the top edges of the implant, the rim surface joining upper edges of the inner and outer surfaces; providing at least one elevated portion and at least one non-elevated portion on the rim surface.

The method of the present invention further comprises at least one of the following steps to adapt the shape of the transitional area to the geometry of the neck: providing the at least one elevated portion with a concave transitional area at each end of the at least one elevated portion, the transitional area connecting a top surface of the at least one elevated portion and the non-elevated portion of the rim surface; forming the elevated portion on approximately one third or less of the circumferential rim surface; and/or providing the at least one elevated portion with a concave inner surface that is a continuation of the inner surface of the cavity of the implant.

The concave transitional area may be formed by a milling cutter.

Experiments were performed in order to evaluate the stability against dislocation and allowed range of motion of the acetabular liner of the present invention compared to that of a standard non-elevated acetabular liner. The liner of the present invention and the standard liner were both made of UHMW polyethylene. Also, a conventional endoprosthetic assembly available on the market was used.

In the below experiments the range of motion with stem antetorsion (AT) 0° of the first embodiment of the liner according to the present invention was determined. The result is shown in diagram (a) :

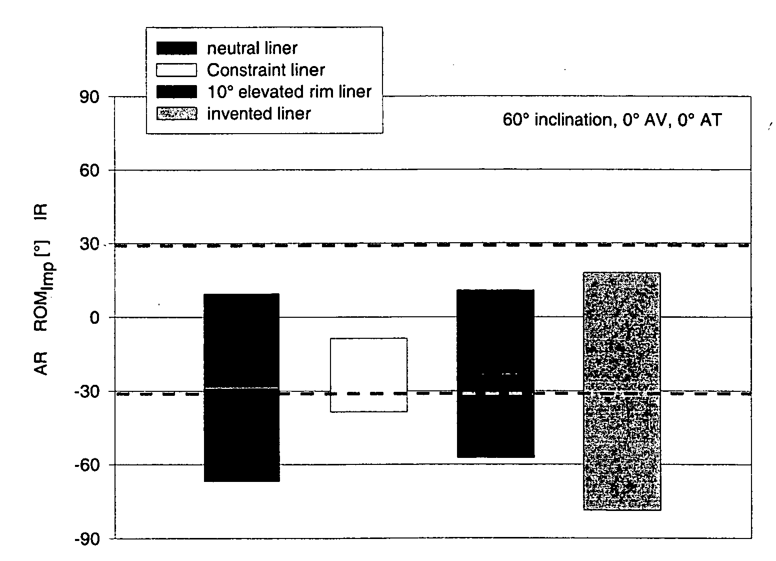

### Diagram (a):

Diagram (a) shows an overall range of motion until impingement (ROM_{Imp}) for the internal und external rotation movement in combination with 90° flexion and 0° adduction, wherein different commercial liner designs are used (neutral, 10° elevated rim liner and constraint liner (without a metallic ring) compared with the invented acetabular liner (in case of backwards pivoted position of 45°).

The implant position was: 60° cup inclination and 0° cup anteversion (AV), stem antetorsion (AT) 0°.

The columns in said diagram show the impingement-free interval in the internal rotation movements for the femoral stem (up to ± 90°) and the dashed lines the physiological range of motion for above-mentioned movements.

Further to the range of motion with stem antetorsion (AT) 0° the range of motion till dislocation was determined as shown in diagram (b) :

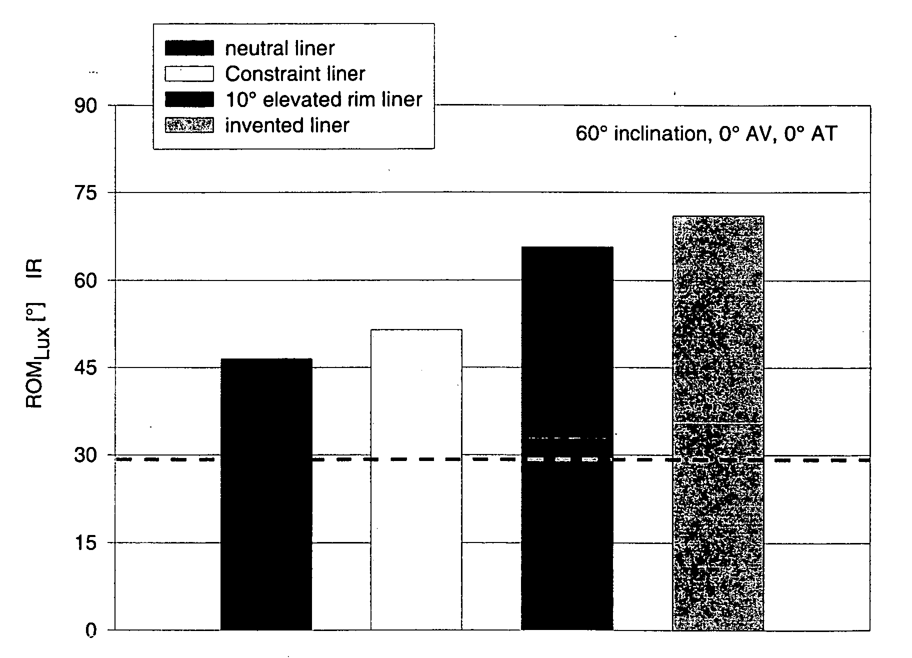

### Diagram (b):

Diagram (b) shows the overall range of motion until dislocation (ROM_{Lux}) for the internal rotation movement in combination with 90° flexion and 0° adduction, wherein different commercial liner designs are used (neutral, 10° elevated rim liner and constraint liner (without a metallic ring) compared to the invented acetabular liner (in case of backwards pivoted position of 45°).

The implant position was: 60° cup inclination and 0° cup anteversion (AV), stem antetorsion (AT) 0°.

The columns in diagram (b) show the impingement-free interval in the internal rotation movements for the femoral stem (up to ± 90°) and the dashed lines the physiological of motion for above-mentioned movements.

As a further measure of range the dislocation stability, the rotational moment associated with subluxation of the femoral head from the liner was measured as shown in diagram (c) :

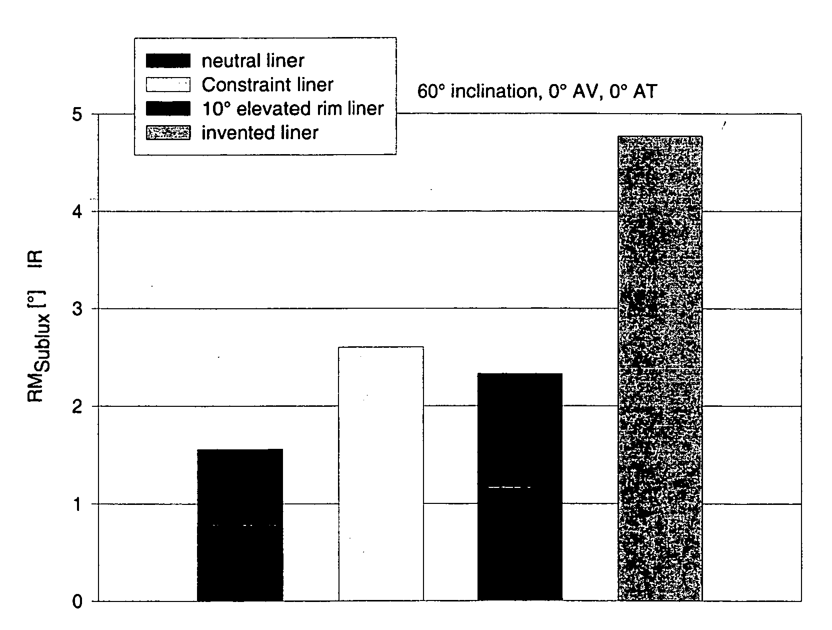

### Diagram (c):

Diagram (c) shows a maximum resisting moment in subluxation for the internal rotation movement in combination with 90° flexion and 0° adduction using different commercial liner designs (neutral, 10° elevated rim liner and constraint liner (without a metallic ring) compared to the invented acetabular liner (in case of backwards pivoted position of 45°)).

The implant position was: 60° cup inclination and 0° cup anteversion (AV), stem antetorsion (AT) 0°.

The experimental results indicated no significant difference in the range of motion with stem antetorsion (AT) 0° allowed by the standard non-elevated liner and that allowed by the liner of the present invention in the case of adequate orientation of the liner within the acetabular cup, i.e. proper position of the elevated rim portion.

Further, the experimental results indicated that the acetabular liner of the present invention provided greater stability of the femoral head than the standard liner in typical movements of the femoral component which normally cause dislocation.

The present invention is not limited to the specific illustrated embodiments. Moreover, the present invention is realised by the features of the claims and any obvious modifications thereof.

## Claims

1. An acetabular liner for receiving a femoral head fixed on a stem component comprising:
an outer surface;
an inner surface being concave and forming a cavity adapted to receive the femoral head; and
a rim surface located circumferentially along the top edge of the liner and joining upper edges of the inner and outer surfaces, the rim surface having at least one elevated portion with a transitional area at each end and at least one non-elevated portion, the transitional area connecting a top surface of the elevated portion and the non-elevated portion of the rim surface, wherein at least a portion of the transitional area is concave for allowing an increased range of motion of the stem component relative to the acetabular liner.

2. An acetabular liner for receiving a femoral head fixed on a stem component comprising:
an outer surface;
an inner surface being concave and forming a cavity adapted to receive the femoral head of the stem component; and
a rim surface located circumferentially along the top edge of the liner and joining upper edges of the inner and outer surfaces, the rim surface having at least one elevated portion and at least one non-elevated portion, wherein the elevated portion forms approximately one third or less of the circumferential rim surface.

3. An acetabular liner for receiving a femoral head fixed on a stem component comprising:
an outer surface;
an inner surface being concave and forming a cavity adapted to receive the femoral head of the stem component; and
a rim surface located circumferentially along the top edge of the liner and joining upper edges of the inner and outer surfaces, the rim surface having at least one elevated portion and at least one non-elevated portion, the elevated portion having a concave inner surface that is a continuation of the inner surface of the cavity of the liner for securely containing the femoral head of the stem component in a snap fit.

4. An acetabular liner according to at least any two of the preceding claims.

5. The acetabular liner according to any of claims 1 to 4, wherein the rim surface extends at least in part above the hemisphere of the cavity.

6. The acetabular liner according to claim 5, wherein the non-elevated portion of the rim surface has a bevelled surface.

7. The acetabular liner according to claim 6, wherein the non-elevated portion of the rim surface is so bevelled to extend approximately to the hemisphere of the cavity such that, when the stem component is moved to an extreme position, a femoral neck of the stem component first contacts an inner edge of the bevelled surface and then contacts the entire bevelled surface.

8. The acetabular liner according to claim 6, wherein the bevelled surface of the non-elevated portion is so formed that a snap fit between the femoral head and the liner is provided over the entire circumference of the cavity of the liner.

9. The acetabular liner according to any one of the preceding claims, wherein the rim surface has substantially one elevated portion.

10. The acetabular liner according to any one of the preceding claims, wherein the shape of the transitional area corresponds to the cross-sectional shape of a neck attached to the femoral head of the stem component.

11. The acetabular liner according to any of claims 1 to 10, wherein the transitional area is curved with a radius of curvature, preferably the radius of curvature being greater than the radius of a neck attached to the femoral head of the stem component.

12. The acetabular liner according to any of claims 1 to 11, further comprising means for attaching the liner securely to an acetabular cup component.

13. A method of manufacturing an implant, in particular an acetabular liner according to any one of the preceding claims, comprising the steps of :
providing an outer surface;
providing an inner surface being concave;
forming a cavity using the inner surface, the cavity being adapted to receive a head fixed on a femoral stem component;
providing a rim surface circumferentially along the top edges of the implant, the rim surface joining upper edges of the inner and outer surfaces;
providing at least one elevated portion and at least one non-elevated portion on the rim surface;
the method comprising at least one of the following steps:
providing the at least one elevated portion with a concave transitional area at each end of the at least one elevated portion, the transitional area connecting a top surface of the at least one elevated portion and the non-elevated portion of the rim surface;
forming the elevated portion on approximately one third or less of the circumferential rim surface;
providing the at least one elevated portion with a concave inner surface that is a continuation of the inner surface of the cavity of the implant.

14. The method according to claim 13, wherein the concave transitional area is formed by a milling cutter.

15. An endoprosthetic assembly for total hip joint replacement comprising:
the acetabular liner according to any one of claims 1 to 12;
an acetabular cup component adapted to receive and connect with the acetabular liner; and
a femoral component comprising a head, neck and stem, wherein the head is adapted to articulate within the cavity of the acetabular liner.
